# EUROPEAN PATENT APPLICATION

(11) **EP 4 663 744 A1**
(43) Date of publication of application: **17.12.2025**
(21) Application number: 24182262.6
(22) Date of filing: 14.06.2024
(51) Int. Cl.: C12M 3/06, C12M 1/42, C12M 3/00

(54) **ORGAN CHIP ASSEMBLY FOR SIMULATING PHYSIOLOGICAL BARRIER ENVIRONMENT**

(71) Applicant: Taipei Medical University, Taipei City 110 (TW)
(72) Inventor: TSENG, How, 110301 TAIPEI CITY (TW); FAN, Yu-Jui, 110301 TAIPEI CITY (TW); CHEN, Shu-Mei, 110301 TAIPEI CITY (TW)
(74) Representative: Lavoix

(57) **Abstract**

An organ chip assembly for simulating physiological barrier environment provided. The organ chip assembly includes a first microfluidic component having a first microfluidic channel, a second microfluidic component having a second microfluidic channel, wherein the second microfluidic channel is configured to receive a membrane and a third microfluidic component having a third microfluidic channel. The first microfluidic component, the second microfluidic component, and the third microfluidic component are configured to be combined. When combined, the second microfluidic component is positioned between the first microfluidic component and the third microfluidic component, such that the first microfluidic channel of the first microfluidic component faces the second microfluidic component and comprises a first portion configured to substantially align with and connect to the second microfluidic channel of the second microfluidic component, and the third microfluidic channel of the third microfluidic component faces the second microfluidic component and comprises a second portion configured to substantially align with and connect to the second microfluidic channel of the second microfluidic component.

## Description

### BACKGROUND

### 1. Field of the Disclosure

The instant disclosure relates to the field of microfluidic system and biomedical technology, specifically relating to an organ-on-chip and method for simulating physiological barrier environment.

### 2. Description of Related Art

The brain is one of the most vital and complex organs in the human body, characterized by its high metabolic demands and inherent fragility. Neurological disorders, such as ischemic stroke, epilepsy, and Alzheimer's disease, significantly impact patients' quality of life. Effective treatment of these conditions is often hindered by the presence of the blood-brain barrier (BBB), a selective permeability barrier that restricts many therapeutic agents from reaching brain tissue.

The BBB protects the brain from harmful substances in the bloodstream while allowing essential nutrients and gases to pass through. However, this protective function complicates the delivery of drugs intended to treat central nervous system (CNS) diseases. Many therapeutic agents, including large molecules and certain small molecules, cannot easily cross the BBB, resulting in insufficient drug concentrations in the brain and suboptimal therapeutic outcomes.

In vitro models that simulate the BBB environment have become essential tools for developing CNS-targeted therapies. These models allow for the examination of drug permeability and transport mechanisms across the BBB, facilitating the screening and optimization of potential therapeutic agents. Typically, they involve co-culturing various brain cells, such as endothelial cells, pericytes, and astrocytes, on microfluidic platforms or membrane-based systems.

The development of these in vitro models is crucial for advancing our understanding of drug transport across the BBB and improving treatments for neurological disorders. By accurately representing the BBB in a laboratory setting, these models can accelerate the development of new CNS therapies, enhancing the quality of life for patients with debilitating brain diseases.

### SUMMARY

According to one example embodiment of the instant disclosure, an organ chip assembly includes a first microfluidic component having a first microfluidic channel, a second microfluidic component having a second microfluidic channel, wherein the second microfluidic channel is configured to receive a membrane and a third microfluidic component having a third microfluidic channel. The first microfluidic component, the second microfluidic component, and the third microfluidic component are configured to be combined. When combined, the second microfluidic component is positioned between the first microfluidic component and the third microfluidic component, such that the first microfluidic channel of the first microfluidic component faces the second microfluidic component and comprises a first portion configured to substantially align with and connect to the second microfluidic channel of the second microfluidic component, and the third microfluidic channel of the third microfluidic component faces the second microfluidic component and comprises a second portion configured to substantially align with and connect to the second microfluidic channel of the second microfluidic component.

According to another example embodiment of the instant disclosure, an organ chip assembly comprises a microfluidic apparatus and an enclosure. The microfluidic apparatus comprises an upper microfluidic channel, a lower microfluidic channel and a middle microfluidic channel between the upper microfluidic channel and the lower microfluidic channel and configured to be in fluid communication with the upper microfluidic channel and the lower microfluidic channel. The middle microfluidic channel is configured to receive a membrane. When the membrane is received in the middle microfluidic channel, a first fluid in the upper microfluidic channel flows over an upper surface of the membrane, and a second fluid in the lower microfluidic channel flows over a lower surface of the membrane. The enclosure is configured to secure and receive the microfluidic apparatus.

According to another example embodiment of the instant disclosure, a method for simulating a physiological barrier environment comprises: providing a membrane, wherein a first surface of the membrane and a second surface opposite the first surface are both populated with cells; providing a microfluidic apparatus, wherein the microfluidic apparatus comprises an upper microfluidic channel, a middle microfluidic channel and a lower microfluidic channel; arranged the membrane within the middle microfluidic channel; providing a first fluid into the upper microfluidic channel, wherein the first fluid immerses the cells on the first surface of the membrane; and providing a second fluid into the lower microfluidic channel, wherein the second fluid immerses the cells on the second surface of the membrane.

In order to further understanding of the instant disclosure, the following embodiments are provided along with illustrations to facilitate appreciation of the instant disclosure; however, the appended drawings are merely provided for reference and illustration, and do not limit the scope of the instant disclosure.

### BRIEF DESCRIPTION OF THE DRAWINGS

FIG. 1A is a schematic perspective view of an organ chip assembly in accordance with an embodiment of the instant disclosure.
FIG. 1B is an exploded perspective view of an organ chip assembly in accordance with an embodiment of the instant disclosure.
FIG. 2A is an exploded perspective view of a microfluidic apparatus of an organ chip assembly in accordance with an embodiment of the instant disclosure.
FIG. 2B is another exploded perspective view of a microfluidic apparatus of an organ chip assembly in accordance with an embodiment of the instant disclosure.
FIG. 3 is an exploded perspective view of an enclosure and a probe device of an organ chip assembly in accordance with an embodiment of the instant disclosure.
FIG. 4A, FIG. 4B. FIG. 4C, FIG. 4D and FIG. 4E illustrate a method of simulating a physiological barrier environment in accordance with an embodiment of the instant disclosure.

### DETAILED DESCRIPTION

The following disclosure provides for many different embodiments, or examples, for implementing different features of the provided subject matter. Specific examples of components and arrangements are described below to explain certain aspects of the present disclosure. These are, of course, merely examples and are not intended to be limiting. For example, the formation of a first feature over or on a second feature in the description that follows may include embodiments in which the first and second features are formed or disposed in direct contact, and may also include embodiments in which additional features are formed or disposed between the first and second features, such that the first and second features are not in direct contact. In addition, the present disclosure may repeat reference numerals and/or letters in the various examples. This repetition is for the purpose of simplicity and clarity and does not in itself dictate a relationship between the various embodiments and/or configurations discussed.

As used herein, spatially relative terms, such as "beneath," "below," "above," "over," "on," "upper," "lower," "left," "right," "vertical," "horizontal," "side" and the like, may be used herein for ease of description to describe one element or feature's relationship to another element(s) or feature(s) as illustrated in the figures. The spatially relative terms are intended to encompass different orientations of the device in use or operation in addition to the orientation depicted in the figures. The device may be otherwise oriented (rotated 90 degrees or at other orientations) and the spatially relative descriptors used herein may likewise be interpreted accordingly. It should be understood that when an element is referred to as being "connected to" or "coupled to" another element, it may be directly connected to or coupled to the other element, or intervening elements may be present.

Present disclosure provides a new type of sheet-like cell-integrated microfluidic chip that can simulate the physiological environment of the vascular system, respiratory system and/or blood-brain barrier. It is intended for research on the absorption mechanisms of targeted drugs (such as organ-, tissue- and brain-targeted drugs), thereby significantly reducing the need for extensive animal experiments in the development of medications. By modeling the dynamics of fluid flow and the forces exerted on vessel walls, it is possible to study phenomena such as wall deformation, arterial compliance, and the propagation of pressure waves in the cardiovascular system. These models are able to better understand the properties of blood vessels in the human body, enabling the development of more personalized or effective diagnostic tools and treatment strategies tailored to patients.

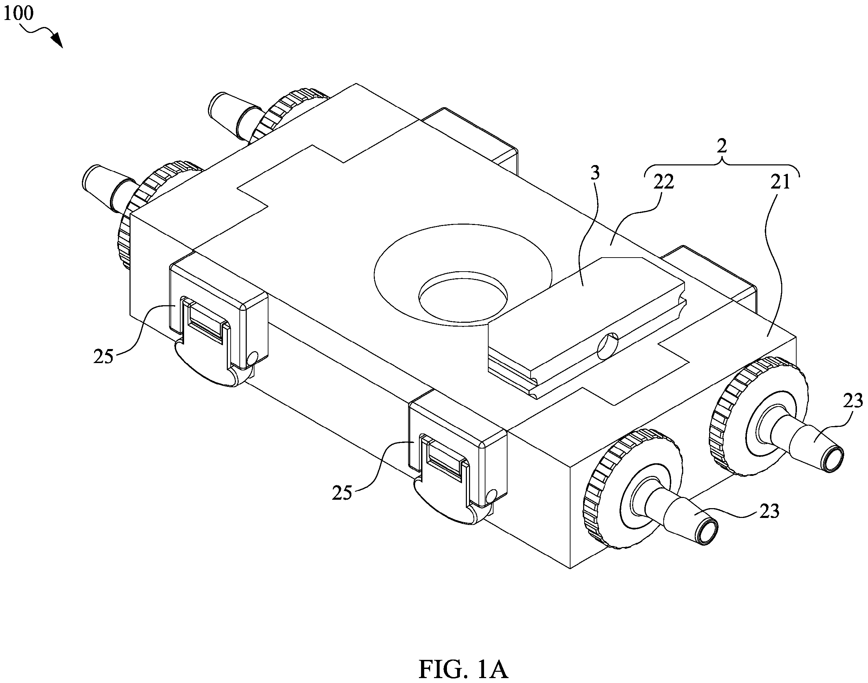

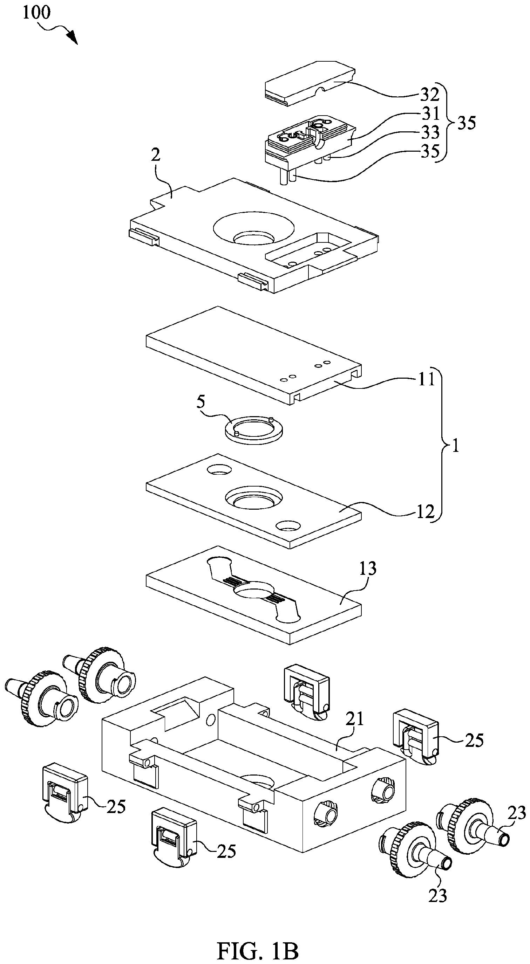

FIG. 1A is a schematic perspective view of an organ chip assembly 100 in accordance with an embodiment of the instant disclosure. FIG. 1B is an is exploded perspective view of the organ chip assembly 100 in accordance with an embodiment of the instant disclosure. Referring to FIG. 1A and FIG. 1B, the organ chip assembly 100 may include a microfluidic apparatus 1, an enclosure 2 and a probe device 3. In some embodiments of the present disclosure, the organ chip assembly 100 is configured to simulate a physiological barrier environment. In some embodiments of the present disclosure, the physiological barrier environment includes a Blood Brain Barrier (BBB) environment.

The microfluidic apparatus 1 may include an upper microfluidic component 11, a middle microfluidic component 12 and a lower microfluidic component 13. As shown in FIG. 1B, a membrane 5 may be mounted to the middle microfluidic component 12, and the upper microfluidic component 11 and the lower microfluidic component 13 may clamp the middle microfluidic component 12 from the top and bottom, so that the upper microfluidic component 11, the middle microfluidic component 12, and the lower microfluidic component 13 are stacked and combined to form the microfluidic apparatus 1, with the middle microfluidic component 12 positioned between the upper microfluidic component 11 and the lower microfluidic component 13. In some embodiments of the present disclosure, the upper microfluidic component 11, the middle microfluidic component 12 and the lower microfluidic component 13 may be primarily made of polydimethylsiloxane (PDMS), a material known for its excellent biocompatibility and moldability. Therefore, when the upper microfluidic component 11, the middle microfluidic component 12 and the lower microfluidic component 13 are stacked and combined to form the microfluidic apparatus 1, they may exhibit good sealing properties between each other. However, they may not be fixed in place, leading to potential movement between of them. Thus, the enclosure 2 is required to secure the microfluidic apparatus 11 formed by the stacking and combining of the upper microfluidic component 11, the middle microfluidic component 12 and the lower microfluidic component 13.

The microfluidic apparatus 1 may include an upper microfluidic component 11, a middle microfluidic component 12, and a lower microfluidic component 13. As shown in FIG. 1B, a membrane 5 may be mounted to the middle microfluidic component 12, and the upper microfluidic component 11 and the lower microfluidic component 13 may clamp the middle microfluidic component 12 from the top and bottom, forming a cohesive unit. This configuration ensures that the upper microfluidic component 11, the middle microfluidic component 12, and the lower microfluidic component 13 are stacked and combined to form the microfluidic apparatus 1, with the middle microfluidic component 12 positioned between the upper microfluidic component 11 and the lower microfluidic component 13.

In some embodiments of the present disclosure, the upper microfluidic component 11, the middle microfluidic component 12, and the lower microfluidic component 13 may be primarily made of polydimethylsiloxane (PDMS), a material known for its excellent biocompatibility and moldability. PDMS is a silicon-based organic polymer widely used in biomedical and microfluidic applications due to its flexibility, optical transparency, and ease of fabrication. The use of PDMS allows for the creation of complex microchannel structures that can accurately replicate physiological conditions, essential for studying cellular behaviors and drug interactions in a controlled environment.

Therefore, when the upper microfluidic component 11, the middle microfluidic component 12, and the lower microfluidic component 13 are stacked and combined to form the microfluidic apparatus 1, they exhibit good sealing properties between each other. These good sealing properties are critical to prevent any leakage of fluids, which is essential for maintaining the integrity of the simulated physiological environment within the microfluidic apparatus. However, due to PDMS's inherent flexibility, the components may not be fixed in place, leading to potential movement between them. Such movement can disrupt the precise alignment and function of the microfluidic channels, affecting the experimental outcomes.

Thus, the enclosure 2 is required to secure the microfluidic apparatus 1 formed by the stacking and combining of the upper microfluidic component 11, the middle microfluidic component 12, and the lower microfluidic component 13. The enclosure provides structural stability and ensures that the components remain in their correct positions during operation. This stability is crucial for conducting accurate and reproducible experiments, as even minor shifts in the microfluidic components can affect the flow dynamics and the interactions between different cell types within the apparatus. The enclosure also helps to protect the microfluidic apparatus from external physical damage and contamination, further enhancing the reliability and longevity of the device.

Referring to FIG. 1B, the enclosure 2 may include a main body 21 and a cover 22. The main body 21 of the enclosure 2 may include an inner space 210 designed to house the microfluidic apparatus 1 securely. Once the microfluidic apparatus 1 is placed within the inner space 210, the cover 22 can be secured using fasteners 25, ensuring the apparatus is firmly held in place. This secure encapsulation is crucial for maintaining the integrity of the microfluidic system, preventing leaks or shifts during experimental procedures. The enclosure 2 is primarily made of titanium alloy, polyethylene (PE), polypropylene (PP), polyvinyl chloride (PVC), polystyrene (PS), polyethylene terephthalate (PET), acrylonitrile butadiene styrene (ABS) or polyolefin chosen for its high biocompatibility and durability. The above listed materials are widely used in biomedical applications due to their strength, resistance to corrosion, and ability to integrate well with biological tissue.

The enclosure 2 may also include a plurality of fluid connectors 23. These connectors are configured to guide external liquids into the microfluidic apparatus 1 and direct liquids from within the microfluidic apparatus 1 to the outside. This precise control over fluid flow is essential for replicating the dynamic environment of the BBB. By introducing various fluids, such as cell culture media, drugs, or other reagents, researchers can simulate different biological scenarios and study their effects on the cells within the microfluidic apparatus. The ability to control fluid dynamics is critical for maintaining the desired microenvironment and ensuring the reproducibility of experimental results.

Referring to FIG. 1A and FIG. 1B, the probe device 3 is configured to match the cover 22 of the enclosure 2. The probe device may include a probe box 31, an upper cover 32 configured to cover the probe box 31 and a plurality of electrode probes 33 and 35 extending from the probe box 31. When the probe device 3 is mounted to the cover 22 of the enclosure 2, the electrode probes 33 and 35 may extend into the microfluidic apparatus 1. This allows the probe device 3 to perform signal measurements measurements on the fluids within the microfluidic apparatus 1. The signal measuremnts include, but are not limited to, , for example, electrical signal (such as Trans-Epithelial Electric Resistance (TEER)), optical signal, thermal signal and vibration signal measurements.

In one embodiment, TEER measurements are a key indicator of barrier function, providing real-time monitoring of the electrical resistance across the cellular layers. This is particularly important for studying the BBB, as it helps in understanding how different compounds affect the barrier's permeability and integrity. The probe device 3 enables precise measurements, facilitating detailed studies on drug delivery, transport mechanisms, and the impact of various pathological conditions on the BBB. This functionality is essential for developing new therapeutic strategies and improving drug formulations aimed at targeting the central nervous system.

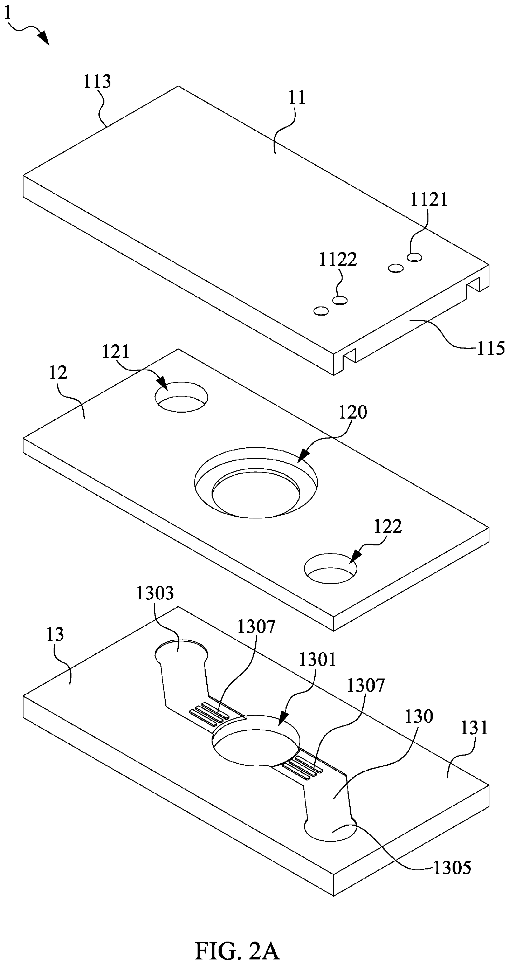

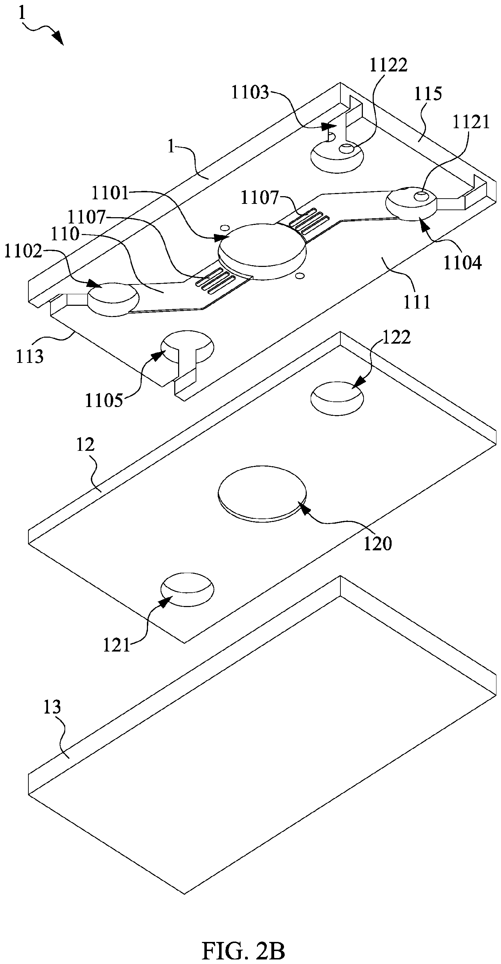

FIG. 2Ais an exploded perspective view of the microfluidic apparatus 1 of an organ chip assembly 100 in accordance with an embodiment of the instant disclosure. FIG. 2B is another exploded perspective view of a microfluidic apparatus 1 of an organ chip assembly 100 in accordance with an embodiment of the instant disclosure. As above-mentioned, the microfluidic apparatus 1 may include the upper microfluidic component 11, the middle microfluidic component 12, and the lower microfluidic component 13. Further, the upper microfluidic component 11, the middle microfluidic component 12, and the lower microfluidic component 13 may be stacked and combined to form the microfluidic apparatus 1. The upper microfluidic component 11, the middle microfluidic component 12 and the lower microfluidic component 13 may be primarily made of polydimethylsiloxane (PDMS). Polydimethylsiloxane (PDMS) exhibits excellent biocompatibility, making it suitable for medical and bioengineering applications. It possesses high elasticity and flexibility, allowing it to be easily processed into various shapes and structures. PDMS also has good gas permeability, enabling effective oxygen and carbon dioxide exchange, which is ideal for cell culture and microfluidic devices. Its transparency facilitates observation and imaging, and its chemical inertness, low toxicity, and compatibility with most chemicals and biological materials further enhance its versatility in various applications.

Referring to FIG. 2A and FIG. 2B, the upper microfluidic component 11 may include a lower surface 111, which may face the middle microfluidic component 12 when the upper microfluidic component 11, the middle microfluidic component 12, and the lower microfluidic component 13 are combined with each other. The upper microfluidic component 11 may include an upper microfluidic channel 110 formed on the lower surface 111. That is, the upper microfluidic channel 110 may also face the middle microfluidic component 12 when the upper microfluidic component 11, the middle microfluidic component 12, and the lower microfluidic component 13 are combined with each other. The upper microfluidic channel 110 may include a fluid inlet 1102 and a fluid outlet 1104. The fluid inlet 1102 may be positioned on one side 113 of the upper microfluidic component 11, while the fluid outlet 1104 may be positioned on the opposite side 115 of the upper microfluidic component 11. That is, a fluid may be introduced into upper microfluidic channel 110 through the fluid inlet 1102 from the side 113 of the upper microfluidic component 11 and may be discharged from the side 115 of the upper microfluidic component 11 through the fluid outlet 1104.

The upper microfluidic channel 110 may include a circular recessed portion 1101. The circular recessed portion 1101 may be substantially located in the midsection of a fluid path formed by the upper microfluidic channel 110. A depth of the circular recessed portion 1101 may be substantially greater than that of the other parts of the upper microfluidic channel 110. Moreover, the circular recessed portion 1101 may substantially match a shape of the membrane 5 mounted to the middle microfluidic component 12. Thus, when the upper microfluidic component 11, the middle microfluidic component 12, and the lower microfluidic component 13 are combined with each other, the circular recessed portion 1101 may cover an upper surface of the membrane 5 mounted to the middle microfluidic component 12 and the fluid passing through the circular recessed portion 1101 of the upper microfluidic channel 110 may flow over the upper surface of the membrane 5.

Further, the upper microfluidic channel 110 may include a plurality of barriers 1107. Each of the barriers 1107 may substantially extend along a flow direction of the fluid flowing within the upper microfluidic channel 110. Moreover, the barriers 1107 may be positioned adjacent the circular recessed portion 1101. The barriers 1107 is configured to disrupt the flow of the fluid flowing within the upper microfluidic channel 110. That is, the barriers 1107 may simulate the shear stress and pulsatile micro-movements of the brain vessel walls. Since the barriers 1107 may be positioned adjacent the circular recessed portion 1101, the barriers 1107 may create flow conditions for the fluid flowing through the recessed portion 1101, similar to the shear stress and pulsatile flow experienced by endothelial cells in brain blood vessels. In microfluidic systems, the design and structural elements within the channels are crucial for simulating physiological conditions. By incorporating barriers such as baffles or grids, it is possible to create flow conditions similar to the shear stress and pulsatile flow experienced by endothelial cells in brain blood vessels. Shear stress is generated as barriers disrupt laminar flow, creating regions with varying flow velocities, localized high shear zones, and a more uniform shear stress distribution. Additionally, barriers induce pulsatile flow by causing periodic flow oscillations, pressure fluctuations, and flow reversal, simulating the dynamic environment of blood flow driven by the heartbeat. These features are essential for accurately mimicking the mechanical signals and conditions that influence cell behavior in vivo.

Moreover, the upper microfluidic component may further include a fluid inlet 1103 and a fluid outlet 1105. The fluid inlet 1103 may be positioned on the side 115 of the upper microfluidic component 11, while the fluid outlet 1105 may be positioned on the opposite side 113 of the upper microfluidic component 11. The fluid inlet 1103 is configured to introduce a fluid into a lower microfluidic channel 130 of the lower microfluidic component 13, while the fluid outlet 1105 is configured to discharge the fluid from channel 130. Further details regarding the structure of the lower microfluidic channel 130 of the lower microfluidic component 13 will be provided later in the description.

In addition, the upper microfluidic component 11 may include through vias 1121 and 1122 formed on an upper surface 112 of the upper microfluidic component 11 and passing through the upper microfluidic component 11. The through vias 1121 may be in fluid communication with the upper microfluidic channel 110 of the upper microfluidic component 11. In some embodiments of the present disclosure, the through vias 1121 are connected to the fluid outlet 1104. The through vias 1122 may be in fluid communication with the lower microfluidic channel 130 of the lower microfluidic component 13. In some embodiments of the present disclosure, the through vias 1122 are connected to the fluid inlet 1103. That is, the electrode probes 33 and 35 of the probe device 3 may extend into the upper microfluidic channel 110 and the lower microfluidic channel 130 through the through vias 1121 and 1122.

The middle microfluidic component 12 may include a middle microfluidic channel 120. The middle microfluidic channel 120 is configured to be in fluid communication with the upper microfluidic channel 110 of the upper microfluidic component 11 and the lower microfluidic channel 130 of the lower microfluidic component 13. In some embodiments of the present disclosure, the middle microfluidic channel 120 includes a circular opening passing through the middle microfluidic component 12. The opening is configured to receive the membrane 5. That is, the membrane 5 may be mounted to the middle microfluidic channel 120 of the middle microfluidic component 12. When the upper microfluidic component 11, the middle microfluidic component 12, and the lower microfluidic component 13 are stacked and combined, the circular recessed portion 1101 of the upper microfluidic channel 110 of the upper microfluidic component 11 may align with the opening of the middle microfluidic channel 120 of the middle microfluidic component 12 and match of the membrane 5 received in the opening of the middle microfluidic channel 120 of the middle microfluidic component 12. Likewise, a circular recessed portion 1301 of the lower microfluidic channel 130 of the lower microfluidic component 13 may align with the opening of the middle microfluidic channel 120 of the middle microfluidic component 12 and match of the membrane 5 received in the opening of the middle microfluidic channel 120 of the middle microfluidic component 12 as well. Further details regarding the structure of the lower microfluidic channel 130 of the lower microfluidic component 13 will be provided later in the description.

Moreover, the middle microfluidic component 12 may include through holes 121 and 122. The through hole 121 is configured to match and/or connect to the fluid outlet 1105 positioned on the side 113 of the upper microfluidic component 11. The through hole 122 is configured to match and/or connect to the fluid inlet 1103 positioned on the side 115 of the upper microfluidic component 11. When the upper microfluidic component 11, the middle microfluidic component 12, and the lower microfluidic component 13 are stacked and combined, the through hole 121 may be in fluid communication with the fluid outlet 1105 and the through hole 122 may be in fluid communication with the fluid inlet 1103.

Referring to FIG. 2A and FIG. 2B, the lower microfluidic component 13 may include an upper surface 131, which may face the middle microfluidic component 12 when the upper microfluidic component 11, the middle microfluidic component 12, and the lower microfluidic component 13 are combined with each other. The lower microfluidic component 13 may include the lower microfluidic channel 130 formed on the upper surface 131. That is, the lower microfluidic channel 130 may also face the middle microfluidic component 12 when the upper microfluidic component 11, the middle microfluidic component 12, and the lower microfluidic component 13 are combined with each other. The lower microfluidic channel 110 may include end portions 1303 and 1305. The end portion 1303 of the lower microfluidic channel 130 is configured to match the through hole 121 of the middle microfluidic component 12. The end portion 1305 of the lower microfluidic channel 130 is configured to match the through hole 122 of the middle microfluidic component 12. When the upper microfluidic component 11, the middle microfluidic component 12, and the lower microfluidic component 13 are combined with each other, the end portion 1303 of the lower microfluidic channel 130 may be in fluid communication with the through hole 121 of the middle microfluidic component 12 and the end portion 1305 of the lower microfluidic channel 130 may be in fluid communication with the through hole 122 of the middle microfluidic component 12. Moreover, as above-mentioned, the through hole 122 may be in fluid communication with the fluid inlet 1103 and the through hole 121 may be in fluid communication with the fluid outlet 1105. That is, a fluid may be introduced into lower microfluidic channel 130 through the fluid inlet 1103 at the side 115 of the upper microfluidic component 11 and the through hole 122 of the middle microfluidic component 12 and may be discharged from the side 113 of the upper microfluidic component 11 through the through hole 121 of the middle microfluidic component 12 and the fluid outlet 1105 at the side 113 of the upper microfluidic component 11. In addition, since the end portion 1305 of the lower microfluidic channel 130, the through hole 122 and the fluid inlet 1103 are in fluid communication with each other, the electrode probes 35 may extend into the end portion 1305 of the lower microfluidic channel 130 through the through vias 1122 and the through holes 122.

The lower microfluidic channel 130 may include the circular recessed portion 1301. The circular recessed portion 1301 may be substantially located in the midsection of a fluid path formed by the lower microfluidic channel 130. A depth of the circular recessed portion 1301 may be substantially greater than that of the other parts of the lower microfluidic channel 130. Moreover, the circular recessed portion 1301 may substantially match a shape of the membrane 5 mounted to the middle microfluidic component 12. Thus, when the upper microfluidic component 11, the middle microfluidic component 12, and the lower microfluidic component 13 are combined with each other, the circular recessed portion 1301 may cover an\ lower surface of the membrane 5 mounted to the middle microfluidic component 12 and the fluid passing through the circular recessed portion 1301 of the lower microfluidic channel 130 may flow over the lower surface of the membrane 5.

Further, the lower microfluidic channel 130 may include a plurality of barriers 1307. Each of the barriers 1307 may substantially extend along a flow direction of the fluid flowing within the lower microfluidic channel 130. Moreover, the barriers 1307 may be positioned adjacent the circular recessed portion 1301. The barriers 1307 is configured to disrupt the flow of the fluid flowing within the upper microfluidic channel 130. That is, the barriers 1307 may simulate the shear stress and pulsatile micro-movements of the brain vessel walls. In microfluidic systems, the design and structural elements within the channels are crucial for simulating physiological conditions. Since the barriers 1307 may be positioned adjacent the circular recessed portion 1301, the barriers 1307 may create flow conditions for the fluid flowing through the recessed portion 1301, similar to the shear stress and pulsatile flow experienced by endothelial cells in brain blood vessels. By incorporating barriers such as baffles or grids, it is possible to create flow conditions similar to the shear stress and pulsatile flow experienced by endothelial cells in brain blood vessels. Shear stress is generated as barriers disrupt laminar flow, creating regions with varying flow velocities, localized high shear zones, and a more uniform shear stress distribution. Additionally, barriers induce pulsatile flow by causing periodic flow oscillations, pressure fluctuations, and flow reversal, simulating the dynamic environment of blood flow driven by the heartbeat. These features are essential for accurately mimicking the mechanical signals and conditions that influence cell behavior in vivo.

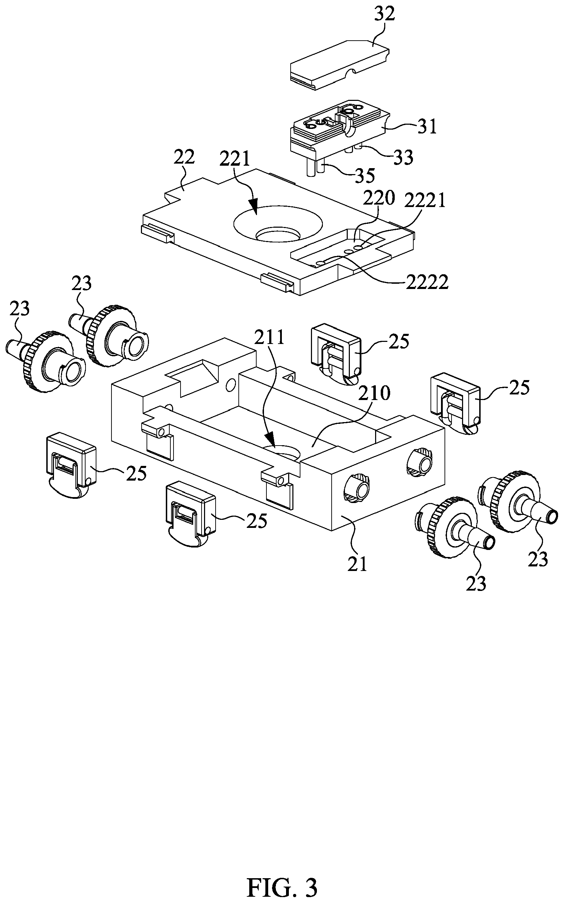

FIG. 3A is an exploded perspective view of the enclosure 2 and the probe device 3 of the organ chip assembly 100 in accordance with an embodiment of the instant disclosure. As shown in FIG. 3, the enclosure 2 may include the main body 21 and the cover 22. The main body 21 of the enclosure 2 may include the inner space 210 which is configured to receive the microfluidic apparatus 1 formed by the stacking and combining of the upper microfluidic component 11, the middle microfluidic component 12, and the lower microfluidic component 13. As above-mentioned, the middle microfluidic component 12 and the lower microfluidic component 13 are primarily made of polydimethylsiloxane (PDMS), a material known for its excellent biocompatibility and moldability. Consequently, when the upper microfluidic component 11, the middle microfluidic component 12, and the lower microfluidic component 13 are stacked and combined to form the microfluidic apparatus 1, they exhibit good sealing properties between each other. However, without proper fixation, there is a risk of relative movement between these components. Therefore, the enclosure 2 is configured to secure the microfluidic apparatus 1, ensuring that the stacked and combined upper, middle, and lower microfluidic components 11, 12 and 13 remain fixed in place, providing additional stability and preventing any potential displacement.

In one embodiment, the enclosure 2 is primarily made of titanium alloy. Titanium alloy possesses excellent mechanical properties and biocompatibility, making it suitable for medical and bioengineering applications. Its high strength-to-weight ratio allows for reduced weight while maintaining structural integrity, which is crucial for platform design. Additionally, titanium alloy exhibits remarkable corrosion resistance, enabling it to withstand humid environments and long-term use within biological systems without degradation, thus enhancing the durability and reliability of the enclosure 2. Furthermore, the low thermal expansion coefficient and superior thermal stability of titanium alloy ensure dimensional stability under temperature variations, which is vital for precise operations and measurements in microfluidic systems. In summary, the use of titanium alloy not only provides mechanical strength and stability but also ensures biocompatibility and long-term durability.

In some embodiments of the present disclosure, the enclosure 2 is equipped with a temperature-controlled water channel within the main body 21, allowing it to connect to an external water circuit for temperature regulation. This ensures that the organ chip assembly 100 maintains a stable temperature at the set physiological temperature or any other desired temperature.

The main body 21 of the enclosure 2 may include a window 211. The cover 22 of the enclosure 2 may include a window 221. When the microfluidic apparatus 1 formed by the stacking and combining of the upper microfluidic component 11, the middle microfluidic component 12, and the lower microfluidic component 13 is encapsulated in the enclosure 2, the windows 211 and 221 may substantially align with the membrane 5 mounted to the middle microfluidic component 12 of the microfluidic apparatus 1. Because the PDMS used to manufacture the microfluidic apparatus 1 may have transparent properties, users may observe the membrane 5 mounted to the middle microfluidic component 12 of the microfluidic apparatus 1 through windows 211 and 221 when using the organ chip assembly 100.

The enclosure 2 may include four fluid connectors 23. These fluid connectors 23 are configured to connect the fluid inlets 1102 and 1104 and fluid outlet 1103 and 1105 of the microfluidic apparatus 1. In some embodiments of the present disclosure, the connector 23 may include a Luer connector. Luer connectors is configured to connect external tubing systems, which include the input and the output of cerebral blood and brain fluids. In some embodiments, the connector 23 connected to the fluid inlet 1102 may serve as the brain fluid input end, and the connector 23 connected to the fluid outlet 1104 may serve as the brain fluid output end. This configuration allows brain fluid to flow within the upper microfluidic channel 110. In some embodiments, the connector 23 connected to the fluid inlet 1103 may serve as the cerebral blood input end, and the connector 23 connected to the fluid outlet 1105 may serve as the cerebral blood output end. This configuration allows cerebral blood to flow within the lower microfluidic channel 110. The primary function of Luer connectors is to ensure stable connections between the internal and external tubing of the microfluidic system.

After the microfluidic apparatus 1 is placed into the inner space 210 of the main body 21 of the enclosure 2, the cover 22 is secured using fasteners 25. These fasteners 25 not only provide a secure fit but also maintain the integrity of the upper, middle and lower microfluidic components 11, 12 and 13, preventing any potential leaks or displacement during operation. The secure attachment of the cover 22 ensures that the organ chip assembly 100 operates under stable conditions, essential for accurate experimental results and reliable performance in various biomedical applications.

The probe device 3 may include the probe box 31, the upper cover 32 configured to cover the probe box 31 and the plurality of electrode probes 33 and 35 extending from the probe box 31. The probe device 3 may be received in a recess 222 formed on an upper surface 220 of the cover 22 of the enclosure 2. The recess 222 may include through vias 2221 and 2222, wherein the through vias 2221 may substantially align with the through vias 1121 of the upper microfluidic component 11 of the microfluidic apparatus 1 and the through vias 2222 may substantially align with the thorough vias 1122 of the upper microfluidic component 11 of the microfluidic apparatus 1. When the probe device 3 is mounted to the enclosure 2 and received in the recess 222 of the cover 22, the electrode probes 33 may pass through the through vias 2221 of the recess 222 of the cover 22 and the through vias 1121 of the upper microfluidic component 11 of the microfluidic apparatus 1 and extend into the upper microfluidic channel 110, and the electrode probes 35 may pass through the through vias 2222 of the recess 222 of the cover 22, the through vias 1122 of the upper microfluidic component 11 of the microfluidic apparatus 1 and the through hole 122 of the middle microfluidic component 12 and extend into the lower microfluidic channel 110.

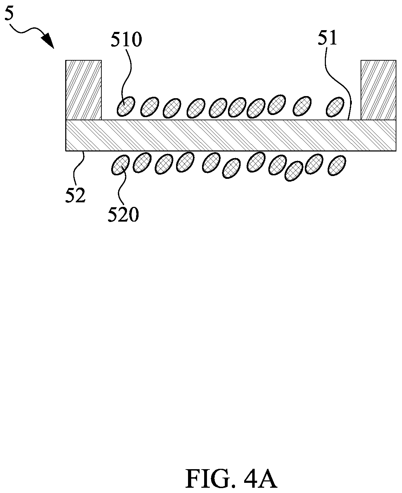

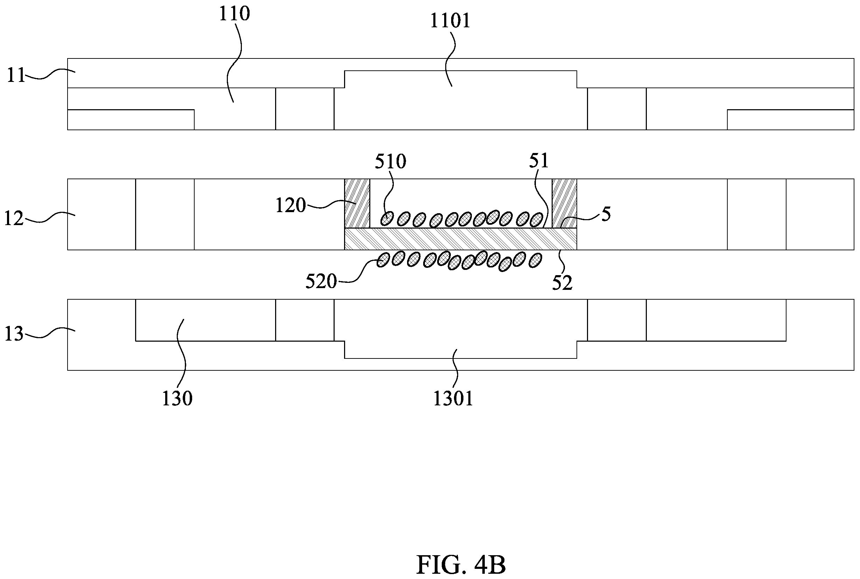

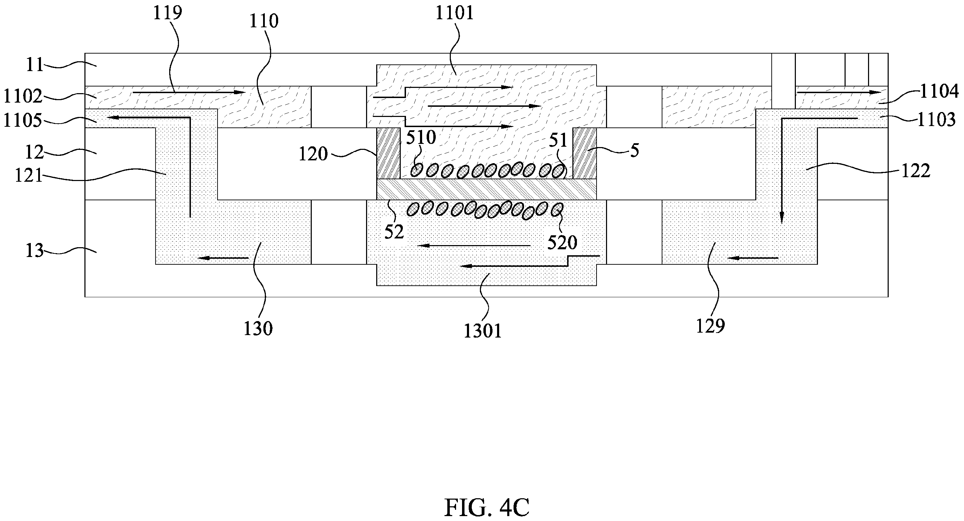

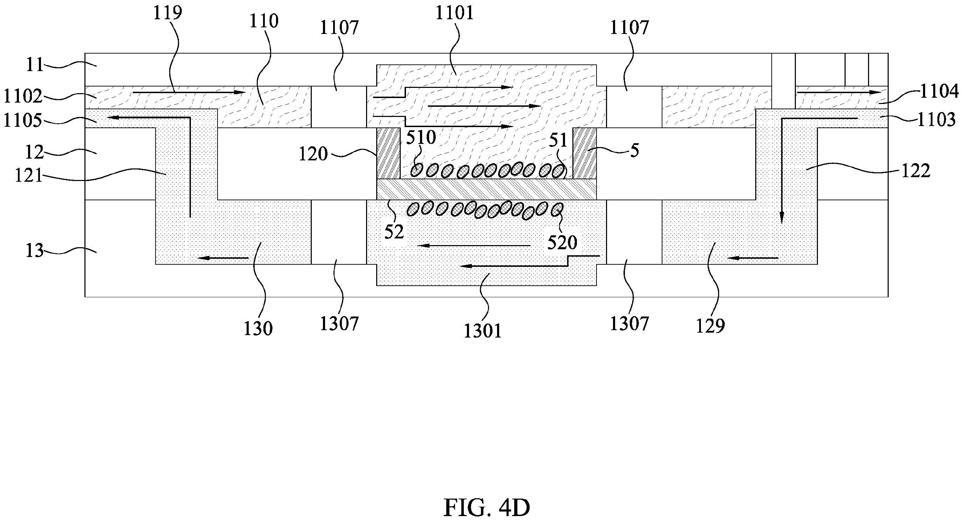

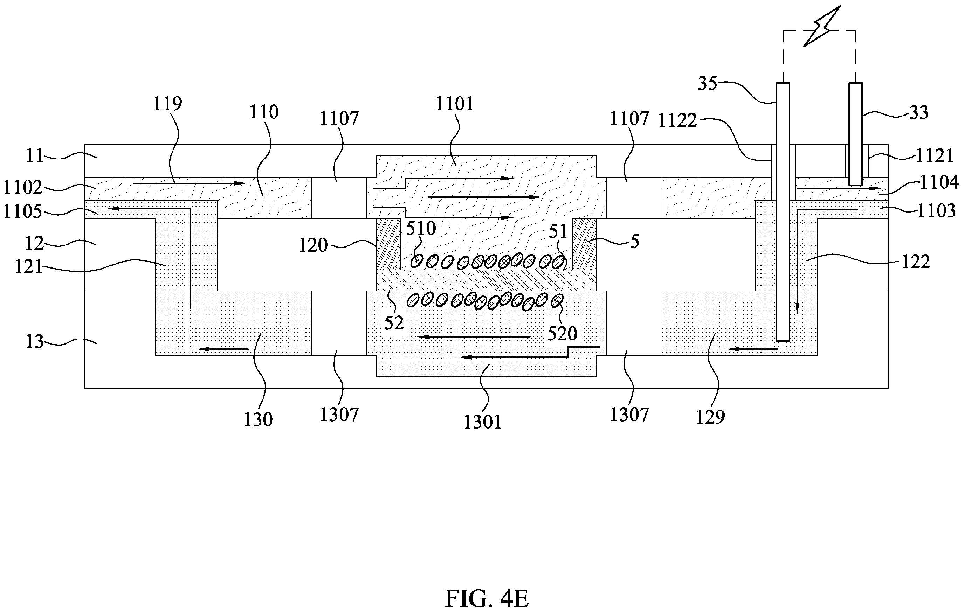

FIG. 4A, FIG. 4B. FIG. 4C, FIG. 4D and FIG. 4E illustrate a method of simulating a physiological barrier environment in accordance with an embodiment of the instant disclosure. Referring to FIG. 4A, the membrane 5 with groups of cells 510 and 520 is provided. As shown in Fig. 4A, the group of cells 510 is cultivated on the upper surface 51 of the membrane 5, and the group of cells 520 is cultivated on the lower surface 52 of the membrane 5. In some embodiments of the present disclosure, the group of cells 510, 520 may include endothelial cells, pericyte and astrocyte. That is, endothelial cells, pericytes, and astrocytes may be co-cultured on the upper surface 51 and lower surface 52 of the membrane 5, thereby constructing a tissue structure similar to that of cerebral blood vessels. In other words, the membrane 5 includes a simulated blood-brain barrier (BBB) environment.

Referring to FIG. 4B, the membrane 5 is mounted to the middle microfluidic channel 120 of the middle microfluidic component 12, and the upper microfluidic component 11, the middle microfluidic component 12, and the lower microfluidic component 13 are stacked and combined to form the microfluidic apparatus 1. The middle microfluidic component 12 is arranged between the upper microfluidic component 11 and the lower microfluidic component 13. The upper microfluidic channel 110 faces the middle microfluidic component 12, and the recessed portion 1101 of the upper microfluidic channel 110 aligns with the middle micro fluidic channel 120 and covers the upper surface 51 of the membrane 5. The lower microfluidic channel 130 faces the middle microfluidic component 12, and the recessed portion 1301 of the lower microfluidic channel 130 aligns with the middle micro fluidic channel 120 and covers the lower surface 52 of the membrane 5.

The upper microfluidic component 11, the middle microfluidic component 12, and the lower microfluidic component 13 are primarily made of polydimethylsiloxane (PDMS), providing good sealing properties when stacked together but making them difficult to secure in place. Therefore, the upper microfluidic component 11, the middle microfluidic component 12, and the lower microfluidic component 13 can be enclosed in enclosure 2, ensuring that they are securely fixed together. After the combined upper, middle and lower microfluidic components 11, 12, and 13 are enclosed in the enclosure 2, an external water circuit may be connected to the temperature-controlled water channel within the main body 21 of the enclosure 2 to maintain the internal temperature of the enclosure 2 at 37°C or at the desired temperature.

Referring to FIG. 4C, a fluid 119 is introduced into the upper microfluidic channel 110, and a fluid 129 is introduced into the lower microfluidic channel 130. In some embodiments of the present disclosure, the fluid 119 includes brain fluids, and the fluid 129 includes cerebral blood. The fluid 119 flows into the upper microfluidic channel 110 from the fluid inlet 1102 and then flows out of the upper microfluidic channel 110 through the fluid outlet 1104. The fluid 119 flows over the upper surface 51 of the membrane 5. The group of cells 510 on the upper surface 51 of the membrane 5 is immersed in the first fluid 119.

Moreover, the fluid 129 flows into the lower microfluidic channel 130 through the fluid inlet 1103 and the through hole 122, and then flows out of the lower microfluidic channel 130 through the through hole 121 and the fluid outlet 1104. The fluid 129 flows over the lower surface 52 of the membrane 5. The group of cells 520 on the lower surface 52 of the membrane 5 is immersed in the first fluid 129. Further, as shown in FIG. 4C, a flow direction of the fluid 119 within the upper microfluidic channel 110 is substantially opposite to a flow direction of the fluid 129 within the lower microfluidic channel 130.

The fluid inlet 1102 and the fluid outlet 1104, which introduce the flow of the fluid 119, may be connected to the fluid connectors 23 of the enclosure 2, and the fluid inlet 1103 and the fluid outlet 1105, which introduce the flow of the fluid 129, may be connected to the fluid connectors 23 of the enclosure 2. The fluid connector 23 may include an Luer connector. The Luer connector is configured to ensure stable connections between the internal channel and external tube.

Referring to FIG. 4D, a test drug is added to the upper microfluidic channel 110 and/or the lower microfluidic channel 130, and the absorption and penetration of the test drug in the blood-brain barrier environment simulated by the membrane 5 is observed. As shown in FIG. 4D, when the fluid 119 flows through the barriers 1107, the barriers 1107 may disrupt the flow of the fluid 119, and when the fluid 129 flows through the barriers 1307, the barriers 1307 may disrupt the flow of the fluid 129. Placing the barriers 1107 in the upper microfluidic channel 110 and the barriers 1307 in the lower microfluidic channel 130 is intended to further simulate and generate shear stress and pulsatile motion similar to those on the cerebral blood vessel walls. In particular, as shown in Fig. 4D, the barriers 1107 in the upper microfluidic channel 110 may create flow conditions for the fluid 119 flowing over the upper surface 51 of the membrane 5, similar to the shear stress and pulsatile flow experienced by endothelial cells in brain blood vessels. Further, the barriers 1307 in the lower microfluidic channel 130 may create flow conditions for the fluid 129 flowing over the lower surface 52 of the membrane 5, similar to the shear stress and pulsatile flow experienced by endothelial cells in brain blood vessels.

Referring to FIG. 4E, the real-time monitoring of Trans-Epithelial Electric Resistance (TEER) is performed. As shown in FIG. 4E, the electrode probe 33 extends into the upper microfluidic channel 110 through the through via 1121 and the electrode probe 35 extends into the lower microfluidic channel 130 through the through hole 122 and the through via 1122. Thus, an electrical resistance across the membrane 5 could be monitored in real time.

Moreover, the enclosure 2 includes windows 211 and 221, and thus the organ assembly 100 be placed on an optical microscope (both visible light and fluorescence microscopes) for observation.

The novel microfluidic device offers significant advantages in the development of brain-targeted drugs, primarily by reducing the reliance on animal testing. This technology substantially minimizes the number of animals needed for drug development, addressing ethical concerns and logistical challenges associated with animal experiments. By providing an accurate in vitro model of the blood-brain barrier (BBB), the device enables researchers to conduct comprehensive drug testing without extensive use of animal models.

Another critical benefit is the real-time monitoring capability of the device. It allows for continuous observation of drug absorption mechanisms within the brain, delivering immediate and precise data on drug efficacy and permeability. This real-time feedback is crucial for optimizing drug formulations and understanding their interactions with the BBB, significantly enhancing the drug development process.

Furthermore, the device accurately simulates the physiological environment of the BBB. By replicating the conditions of brain vasculature, it offers a highly realistic experimental platform that closely matches actual human brain tissue. This enhances the predictive accuracy of in vitro drug testing, ensuring that results are more reflective of in vivo conditions. Consequently, the device improves the reliability and relevance of preclinical drug evaluations, accelerating the development of effective and safe treatments for neurological disorders.

As used herein, the singular terms "a," "an," and "the" may include a plurality of referents unless the context clearly dictates otherwise.

As used herein, the terms "approximately," "substantially," "substantial" and "about" are used to describe and account for small variations. When used in conjunction with an event or circumstance, the terms can refer to instances in which the event or circumstance occurs precisely as well as instances in which the event or circumstance occurs to a close approximation. For example, when used in conjunction with a numerical value, the terms can refer to a range of variation of less than or equal to ±10% of that numerical value, such as less than or equal to ±5%, less than or equal to ±4%, less than or equal to ±3%, less than or equal to ±2%, less than or equal to ±1%, less than or equal to ±0.5%, less than or equal to ±0.1%, or less than or equal to ±0.05%. For example, two numerical values can be deemed to be "substantially" the same or equal if the difference between the values is less than or equal to ±10% of an average of the values, such as less than or equal to ±5%, less than or equal to ±4%, less than or equal to ±3%, less than or equal to ±2%, less than or equal to ±1%, less than or equal to ±0.5%, less than or equal to ±0.1%, or less than or equal to ±0.05%. For example, "substantially" parallel can refer to a range of angular variation relative to 0° that is less than or equal to ±10°, such as less than or equal to ±5°, less than or equal to ±4°, less than or equal to ±3°, less than or equal to ±2°, less than or equal to ±1°, less than or equal to ±0.5°, less than or equal to ±0.1°, or less than or equal to ±0.05°. For example, "substantially" perpendicular can refer to a range of angular variation relative to 90° that is less than or equal to ±10°, such as less than or equal to ±5°, less than or equal to ±4°, less than or equal to ±3°, less than or equal to ±2°, less than or equal to ±1°, less than or equal to ±0.5°, less than or equal to ±0.1°, or less than or equal to ±0.05°.

Additionally, amounts, ratios, and other numerical values are sometimes presented herein in a range format. It is to be understood that such range format is used for convenience and brevity and should be understood flexibly to include numerical values explicitly specified as limits of a range, but also to include all individual numerical values or sub-ranges encompassed within that range as if each numerical value and sub-range were explicitly specified.

While the present disclosure has been described and illustrated with reference to specific embodiments thereof, these descriptions and illustrations do not limit the present disclosure. It should be understood by those skilled in the art that various changes may be made and equivalents may be substituted without departing from the true spirit and scope of the present disclosure as defined by the appended claims. The illustrations may not be necessarily drawn to scale. There may be distinctions between the artistic renditions in the present disclosure and the actual apparatus due to manufacturing processes and tolerances. There may be other embodiments of the present disclosure which are not specifically illustrated. The specification and drawings are to be regarded as illustrative rather than restrictive. Modifications may be made to adapt a particular situation, material, composition of matter, method, or process to the objective, spirit and scope of the present disclosure. All such modifications are intended to be within the scope of the claims appended hereto. While the methods disclosed herein are described with reference to particular operations performed in a particular order, it will be understood that these operations may be combined, sub-divided, or re-ordered to form an equivalent method without departing from the teachings of the present disclosure. Accordingly, unless specifically indicated herein, the order and grouping of the operations are not limitations on the present disclosure.

## Claims

1. An organ chip assembly (100), comprising:
a first microfluidic component (11) having a first microfluidic channel (110);
a second microfluidic component (12) having a second microfluidic channel (120), wherein the second microfluidic channel is configured to receive a membrane (5); and
a third microfluidic component (13) having a third microfluidic channel (130);
wherein the first microfluidic component (11), the second microfluidic component (12), and the third microfluidic component (13) are configured to be combined, and wherein, when combined, the second microfluidic component (12) is positioned between the first microfluidic component (11) and the third microfluidic component (13), such that the first microfluidic channel (110) of the first microfluidic component (11) faces the second microfluidic component (12) and comprises a first portion (1101) configured to substantially align with and connect to the second microfluidic channel (120) of the second microfluidic component (12), and the third microfluidic channel (130) of the third microfluidic component (13) faces the second microfluidic component (12) and comprises a second portion (1301) configured to substantially align with and connect to the second microfluidic channel (120) of the second microfluidic component (12).

2. The organ chip assembly of claim 1, wherein the first microfluidic channel (110) of the first microfluidic component (11) is configured to receive a first fluid (119) and the third microfluidic channel (130) of the third microfluidic component (13) is configured to receive a second fluid (129), and wherein the first fluid (119) is different from the second fluid (129).

3. The organ chip assembly of claim 2, wherein the first microfluidic channel (110) of the first microfluidic component (11) comprises a plurality of first barriers (1107), each of the first barriers (1107) substantially extending along a flow direction of the first fluid (119), and wherein the third microfluidic channel (130) of the third microfluidic component (13) comprises a plurality of second barriers (1307), each of the second barriers (1307) substantially extending along a flow direction of the second fluid (129).

4. The organ chip assembly of claim 3, wherein the plurality of first barriers (1107) is positioned adjacent to the first portion (1101) of the first microfluidic channel (110), and wherein the plurality of second barriers (1307) is positioned adjacent to the second portion (1301) of the third microfluidic channel (130).

5. The organ chip assembly of claim 1, wherein the first microfluidic component (11) comprises a first inlet (1102), a second inlet (1103), a first outlet (1104), and a second outlet (1105), and wherein the second microfluidic component (12) comprises a first through hole (122) and a second through hole (121), wherein the first inlet (1102) and the first outlet (1104) are in fluid communication with the first microfluidic channel (110), wherein the first through hole (122) is substantially aligned with the second inlet (1103) and in fluid communication with the third microfluidic channel (130) of the third microfluidic component (13), and wherein the second through hole (121) is substantially aligned with the second outlet (1105) and in fluid communication with the third microfluidic channel (130) of the third microfluidic component (13).

6. The organ chip assembly of claim 5, wherein the first inlet (1102) and the second outlet (1105) are positioned on a first side (113) of the first microfluidic component (11), and wherein the first outlet (1104) and the second inlet (1103) are positioned on a second side (115) of the first microfluidic component (11), opposite the first side.

7. The organ chip assembly of claim 5, further comprising an enclosure (2) configured to encapsulate the first microfluidic component (11), the second microfluidic component (12) and the third first microfluidic component (13).

8. The organ chip assembly of claim 7, wherein the enclosure comprises four fluid connectors (23), each of the four fluid connectors (23) being in fluid communication with the first inlet (1102), the first outlet (1104), the second inlet (1103), and the second outlet (1105), respectively.

9. The organ chip assembly of claim 7, wherein the enclosure (2) comprises a first via (2221) and a second via (2222), and the first microfluidic component (11) comprises a third via (1121) substantially aligned with the first via (2221) of the enclosure (2) and connected to the first outlet (1104) and a fourth via (1122) substantially aligned with the second via (2222) and connected to the second inlet (1103), and wherein the first via (2221) and the third via (1121) are configured to enable a first electrode probe (33) to extend into the first microfluidic channel (110), and the second via (2222) and the fourth via (1122) are configured to enable a second electrode probe (35) to extend into the third microfluidic channel (130).

10. The organ chip assembly of claim 9, further comprising a probe device (3) with the first electrode probe (33) and the second electrode probe (35), wherein the probe device (3) is configured to match the enclosure (2).

11. The organ chip assembly of claim 1, wherein the first microfluidic component (11), the second microfluidic component (12) and the third microfluidic component (13) comprise polydimethylsiloxane (PDMS) material.

12. The organ chip assembly of claim 7, wherein the enclosure (2) comprises titanium material, polyethylene (PE), polypropylene (PP), polyvinyl chloride (PVC), polystyrene (PS), polyethylene terephthalate (PET), acrylonitrile butadiene styrene (ABS) or polyolefin.

13. The organ chip assembly of claim 7, wherein the enclosure (2) comprise at least one window (211. 221) aligned with the second microfluidic channel (120) of the second microfluidic component (12).

14. A method for simulating a physiological barrier environment, comprising:
providing a membrane (5), wherein a first surface (51) of the membrane (5) and a second surface (52) opposite the first surface are both populated with cells (510 & 520);
providing a microfluidic apparatus (1), wherein the microfluidic apparatus (1) comprises an upper microfluidic channel (110), a middle microfluidic channel (120) and a lower microfluidic channel (130);
arranged the membrane (5) within the middle microfluidic channel (120);
providing a first fluid (119) into the upper microfluidic channel (110), wherein the first fluid (119) immerses the cells on the first surface (51) of the membrane (5); and
providing a second fluid (129) into the lower microfluidic channel (130), wherein the second fluid (129) immerses the cells on the second surface (52) of the membrane (5).

15. The method of claim 17, further comprising:
disrupting a flow of the first fluid (119) in the upper microfluidic channel (110); and
disrupting a flow of the second fluid (129) in the lower microfluidic channel (130).
